Europäisches Patentamt

European Patent Office

Office européen des brevets

(19)

(11) Numéro de publication: **0 072 319**
**B1**

## FASCICULE DE BREVET EUROPÉEN

(12)

(45) Date de publication du fascicule du brevet:
15.01.86

(51) Int. Cl.⁴: **G 01 N 33/569**

(21) Numéro de dépôt: **82401474.0**

(22) Date de dépôt: **04.08.82**

(54) Toxoplasmes colorés en suspension stable et leur application au dépistage de la toxoplasmose.

(30) Priorité: **05.08.81 FR 8115219**

(43) Date de publication de la demande:
**16.02.83 Bulletin 83/7**

(45) Mention de la délivrance du brevet:
**15.01.86 Bulletin 86/3**

(84) Etats contractants désignés:
**BE CH DE IT LI LU NL**

(56) Documents cité:
**EP-A-0 011 716**
**FR-A-1 602 301**
**FR-A-2 226 468**

**ANNALES DE BIOLOGIE CLINIQUE, vol. 28, 1970, pages 411-415; P. COUZINEAU et al.: "Agglutination directe des toxoplasmes. Préparation de l'antigène et examen de 400 sérums"**
**MEDISCHES LABORATORIUM, vol. 27, no. 7, 1974, pages 169-173; Y. PELOUX: "La réaction d'agglutination directe des toxoplasmes"**
**CHEMICAL ABSTRACTS, vol. 78, no. 5, 5 février 1973, page 343, col. 1, no. 27696f, Columbus Ohio (USA); H. THORBURN et al.: "Stable hemagglutinating antigen for detecting toxoplasma antibodies"**
**Science, vol. 106 (1948), pages 615-642**

(73) Titulaire: **LABORATOIRES BIOTROL S.A., 1, rue du Foin, F-75140 Paris Cedex 03 (FR)**

(72) Inventeur: **Rigault, Yolande, 48, allée de la Blancharde, F-91190 Gyf Sur Yvette (FR)**
Inventeur: **Lawny, François, Lotissement de Vieux Moulin, F-60350 Cuise La Motte (FR)**

(74) Mandataire: **Phélip, Bruno, c/o Cabinet Harlé & Phélip 21, rue de la Rochefoucauld, F-75009 Paris (FR)**

## Description

La présente invention concerne le dépistage de la toxomoplasmose. Elle concerne plus particulièrement un réactif pour la détermination de la toxoplasmose par agglutination directe, composé de toxoplasmes colorés en suspension stable dans un milieu liquide et un procédé amélioré pour le dépistage de la toxoplasmose utilisant de tels toxoplasmes et reposant sur une agglutination directe de ceux-ci.

Parmi les tests connus de dépistage de la toxoplasmose, l'agglutination directe est un des plus répandus. Son principe est connu depuis 1965: il consiste à visualiser la présence éventuelle d'anticorps anti-toxoplasmes par l'agglutination d'une suspension de parasites dans une cupule de microtitration. En présence d'anticorps, les parasites, reliés par un réseau de gamma-globulines, forment un voile qui tapisse le fond de la cupule. En l'absence d'anticorps, par contre, ils sédimentent en bouton.

Sur ce principe, le document FR-A-1.602.301 décrit des compositions d'un mélange anti-gamma-globulines fluorescentes auxquelles est ajouté un contre-colorant du type Bleu Evans et qui sont destinées à être utilisées lors des examens microscopiques par immunofluorescence; le contre-colorant a alors pour fonction d'améliorer le contraste de lecture de la fluorescence et de diminuer la fluorescence non spécifique.

L'inconvénient majeur de cette réaction très spécifique est de fatiguer la vue des personnes qui effectuent ces tests. Les toxoplasmes sont pratiquement transparents et les voiles d'agglutination sont pratiquement invisibles. Les boutons de sédimentation exigent un éclairage rasant pour être bien visibles.

De ce fait, nombre de biologistes préfèrent utiliser des hématies sensibilisées avec un extrait antigénique de toxoplasmes. L'hémagglutination ou la sédimentation, peut alors être constatée sans aucune fatigue visuelle. Cependant, pour être spécifique, la réaction nécessite qu'on épuise au préalable les sérums en anticorps anti-hématies, de façon à ne laisser subsister que les anticorps anti-toxoplasmose. Cette technique présente également un autre inconvénient comme elle fait appel à un antigène figuré, réalisé en greffant chimiquement une fraction antigénique solubilisée sur une hématie, la réponse de la réaction d'hémagglutination est moins parallèle à la technique de référence (dite "dyetest") que l'agglutination directe.

Une autre méthode, décrite notamment par Sabin A.B et al dans Science, 108, pages 660-663 (1948) utilise des toxoplasmes vivants, et un colorant qui permet de les visualiser. Lorsque ceux-ci sont mis au contact d'un sérum contenant des anticorps dirigés contre eux, ils sont lysés et donc tués; comme seul le cytoplasme des toxoplasmes vivants est coloré par le bleu de méthylène, il est possible de déduire, après incubation du sérum avec des toxoplasmes vivants puis addition du colorant, la quantité d'anticorps présents dans le sérum de la quantité de cellules colorées. La mise en oeuvre d'une telle méthode est délicate et les auteurs précisent diverses conditions opératoires. La présente invention a pour but un réactif pour le dosage de la toxoplasmose par agglutination directe, composé d'une suspension de toxoplasmes tués, caractérisé en ce qu'ils sont colorés avec un colorant des organites ou composants cellulaires choisi de telle sorte qu'il y ait corservation des propriétés antigéniques des toxoplasmes et que la suspension soit stable dans le temps. La présente invention a pour but de procurer un moyen de dépistage de la toxoplasmose qui allie la spécificité à la lisibilité et permette ainsi de procéder à des tests sûrs sans fatigue visuelle excessive.

On y parvient selon l'invention en rendant l'agglutination directe très facilement lisible grâce à l'utilisation de toxoplasmes tués colorés, en suspension stable, c'est-à-dire d'une suspension de toxoplasmes colorés qui garde ses qualités d'antigénicité et de coloration pendant une longue période, en pratique pendant plusieurs mois.

La réaction d'agglutination est alors aussi nette qu'elle le serait avec des hématies.

Il est bien certain que le principe consistant à colorer des microorganismes est connu depuis longtemps. Cependant, toutes ces colorations ont en commun deux points qui les distinguent de la présente invention, du point de vue tant du choix et de l'agencement que de la finalité des moyens concernés:

- la coloration est toujours extemporanée et est destinée à faciliter un examen microscopique immédiat. La préoccupation de stabilité à long terme de la coloration et de la qualité antigénique de parasite est toujours absente de ce type d'examen.

- la coloration n'a jamais été employée, à la connaissance de la demanderesse, pour visualiser un phénomène d'agglutination d'une suspension de toxoplasmes tués, même extemporanément.

C'est ainsi qu'on emploie classiquement notamment du Bleu Evans pour colorer les toxoplasmes et améliorer par ce biais le contraste de lecture en immuno-fluorescence; mais selon cette technique, qui repose sur une lecture en immuno-fluorescence, les parasites sont déposés sur une lame de microscope et mis à sécher, et on dépose sur la forme sèche du produit une goutte de colorant, on laisse incuber, puis on rince. Cette technique de coloration extemporanée a fondamentalement pour objectif d'améliorer le contraste entre le bord du microorganisme et le centre, lors d'un examen par fluorescence (voir par exemple Ann. Biol. Clin., vol. 28, 1970, pages 411-415).

Selon l'invention, par contre, on prépare des toxoplasmes tués, colorés en suspension stable en milieu liquide et on satisfait à la double exigence de stabilité à long terme de la coloration et de la qualité antigénique du moyen de dépistage de la toxoplasmose ainsi réalisé. Pour ce faire, on traite une suspension du parasite non sèche, en y fixant un colorant selon les techniques connues de l'homme de l'art et appropriées au type de colorant choisi, et on lave pour enlever l'excès de colorant.

Les substances colorantes qui peuvent être mises en oeuvre selon l'invention sont:

- d'une manière générale tous les colorants des protéines, des lipides, des lipo-protéines, des mucopolysaccharides, des glycoprotéines et/ou des acides nucléiques.

- plus particulièrement tous les colorants du cytoplasme, des vacuoles, du noyau et autres organites intracellulaires ou des membranes cellulaires; et

- plus spécifiquement, mais à titre d'exemples uniquement:

le Noir Soudan B (qui est actuellement le colorant qu'on préfère à tous les autres), le Bleu Evans, les sels de tétrazolium, l'acide osmique, l'iode, etc......

Avant de mettre en oeuvre un colorant déterminé conformément à l'invention, il convient de vérifier par un test préalable si ce colorant est approprié ou non. Le test à effectuer est double et peut se définir comme suit:.

Après la réaction de coloration en milieu liquide, les toxoplasmes sont lavés et centrifugés jusqu'à ce que le surnageant des centrifugations soit limpide et incolore. Si les toxoplasmes sont encore colorés, on teste leur qualité antigénique par la réaction bien connue d'agglutination directe:

- les parasites ne doivent pas avoir perdu de sensibilité (même titre d'agglutination pour le meme sérum positif, avant et après coloration),

- ils ne doivent pas avoir perdu de spécificité: les sérums négatifs ne doivent pas donner d'agglutination.

Si ces tests sont satisfaisants, on examine alors la stabilité de la coloration:

- à court terme, en vieillissement accéléré de quelques jours à 37°C,

- à long terme, en vieillissement naturel d'un an à + 4°C.

Le milieu de suspension doit rester suffisamment peu coloré pour permettre une parfaite visualisation des parasites, et les qualités de spécificité et de sensibilité doivent être conservées.

Si tous ces critères sont satisfaits, le colorant testé peut être retenu.

L'invention a donc pour premier objet des toxoplasmes tués colorés en suspension stable en milieu liquide, qui satisfont au double test d'antigénicité, et de stabilité défini ci-dessus.

Selon une variante tout particulièrement préférée, mise en oeuvre selon les méthodes faisant appel à des techniques de fixation de substances entre elles par liaisons covalentes que l'homme de l'art est apte à adapter au cas d'espèce, les toxoplasmes colorés selon l'invention comportent un chromophore fixé par liaison chimique covalente entre des fonctions respectives appropriées dudit chromophore et du toxoplasme.

L'invention a également pour objet l'application de ces toxoplasmes colorés en suspension stable en milieu liquide au dépistage de la toxoplasmose par agglutination directe avec formation d'un voile coloré. La technique d'agglutination directe à laquelle peuvent être soumis les toxoplasmes colorés est décrite, notamment, dans "Agglutination Directe des Toxoplasmes" P.COUZINEAU et H. BAUFINE-DUCROCQ-Ann. Biol. Clin. 28, 411-415 (1970).

L'invention est décrite plus en détail dans les exemples concrets ci-après, qui visent à mieux l'illustrer et ne la limitent aucunement.

**EXEMPLE 1: Coloration du cytoplasme**

Les toxoplasmes sont suspendus dans une solution de Bleu Evans à 25 mg/l, de préférence tamponnée à pH 7,0 par un tampon phosphate 0,02 M contenant 7,5 g/l de chlorure de sodium (P.B.S.).

Après incubation d'une demi-heure à 37°C, les toxoplasmes sont rincés environ 5 fois par centrifugation dans du P.B.S. jusqu'à obtention d'un surnageant incolore.

La richesse de la suspension est alors ajustée à 100.000 parasites/microlitre par dilution dans du P.B.S. contenant un conservateur, par exemple de l'azide de sodium à lg/l. Cette suspension est prête à l'emploi pour les tests dits d'agglutination directe.

**EXEMPLE 2 - Coloration des substances lipidiques**

On prépare une solution de Noir Soudan B dans la proportion suivante:

- Noir Soudan B 0,2 g
- Ethanol 70 ml
- Eau 30 ml

A 5 ml de suspension de toxoplasmes dans du P.B.S., on ajoute 2 ml de cette solution de Noir Soudan B.

Le mélange est mis à incuber 30 minutes à 37°C, puis les toxoplasmes sont rincés comme dans l'exemple 1.

La richesse de la suspension est ajustée de la même façon que dans l'exemple 1 et cette suspension ajustée est également prête à l'emploi pour des tests d'agglutination directe sur toxoplasmes colorés.

**EXEMPLE 3 - Couplage chimique d'un chromophore.**

Cet exemple a pour but de montrer qu'on peut également coupler une substance colorée par une liaison chimique covalente entre le chromophore et le support toxoplasme.

On suspend les parasites dans une solution fraîche à 1 % d'acide périodique dans l'eau, ce qui transforme les alcools vicinaux des sucres en aldéhyde.

Après incubation de 5 minutes, les toxoplasmes sont rincés une fois au P.B.S. On ajoute alors une solution de fuchsine basique préparée de la façon suivante:

- Fuschsine basique 2 g
- Métabisulfite de potassium 4 g
- Acide chlorhydrique 2 N 40 ml
- Eau 400 ml.

On incube 15 minutes à température ambiante.

Les amines sont capables de se coupler aux fonctions aldéhydes créées par l'oxydation à l'acide periodique. On conjugue ainsi la fuchsine au toxoplasme.

On rince 2 fois pendant 5 minutes avec une solution légèrement acide (pH 2,5) de 4 g/l de métabisulfite de potassium dans de l'eau additionnée d'acide chlorhydrique.

On termine par un rinçage au P.B.S. et l'ajustement de la suspension comme décrit dans l'exemple 1. On obtient ainsi une suspension prête à l'emploi pour des tests d'agglutination directe améliorés conformément à l'invention.

**Revendications**

1. Réactif pour le dosage de la toxoplasmose par agglutination directe composé d'une suspension de toxoplasmes tués, caractérisé en ce qu'ils sont colorés avec un colorant des organites ou composants cellulaires choisi de telle sorte qu'il y ait conservation des propriétés antigéniques des toxoplasmes et que la suspension soit stable dans le temps.

2. Réactif selon la revendication 1 caractérisé en ce que le colorant est choisi parmi le Noir Soudan B et le Bleu Evans.

3. Réactif selon la revendication 1, caracterisé en ce que le colorant est un chromophore fixé de façon covalente indirecte au toxoplasme.

4. Réactif selon la revendication 3, caractérisé en ce que le chromophore est la fuchsine et que le toxoplasme est préalablement traité à l'acide periodique.

5. Réactif selon l'une quelconque des revendications précédentes, caractérisé en ce que les toxoplasmes après coloration sont suspendus dans une solution de tampon phosphate 0,02 M contenant 7,5 g/l de chlorure de sodium et un agent conservateur.

**Patentansprüche**

1. Reagenz zur Dosierung der Toxoplasmose durch direkte Agglutination, bestehend aus einer Suspension abgetöteter Toxoplasmen, dadurch gekennzeichnet, daß sie mit einem Farbstoff der Organiten oder Zellenbestandteile gefärbt werden, der derart gewählt wird, daß die antigenen Eigenschaften der Toxoplasmen erhalten bleiben, und daß die Suspension in der Zeit stabilisiert ist.

2. Reagens nach Anspruch 1, dadurch gekennzeichnet, daß der Farbstoff zwischen dem Sudanschwarz B und dem Evansblau gewählt wird.

3. Reagens nach Anspruch 1, dadurch gekennzeichnet, daß der Farbstoff ein kovalentartig mit dem Toxoplasma indirekt gebundener Chromophor ist.

4. Reagenz nach Anspruch 3, dadurch gekennzeichnet, daß der Chromophor Fuchsin ist, und daß das Toxoplasma mit Perjodsaüre vorbehandelt wird.

5. Reagens nach einem der vorhergehenden Ansprüche, dadurch gekennzeichnet, daß die Toxoplasmen nach der Färbung in einer 0,02 M Phosphatpufferlösung mit 0,5 g/l Natriumchlorid und einem Konservierungsstoff suspendiert werden.

**Claims**

1. Reagent for assay of toxoplasmosis by direct agglutination, comprising a suspension of killed toxoplasma, characterised in that the latter are stained with a dye for the organelles or cell components chosen in such a way that the antigenic properties of the toxoplasma are preserved and that the suspension is stable in course of time.

2. Reagent according to Claim 1, characterised in that the dye is chosen from Sudan Black B and Evans Blue.

3. Reagent according to Claim 1, characterised in that the dye is a chromophoric compound indirectly covalently bound to the toxoplasma.

4. Reagent according to Claim 3, characterised in that the chromophoric compound is fuchsin and in that the toxoplasma is treated beforehand with periodic acid.

5. Reagent according to any one of the preceding claims, characterised in that the toxoplasma after staining are suspended in a 0.02 M phosphate buffer containing 7.5 g/l of sodium chloride and a preservative.